# EUROPEAN PATENT APPLICATION

(11) **EP 3 547 321 A1**
(43) Date of publication of application: **02.10.2019**
(21) Application number: 18190613.2
(22) Date of filing: 24.08.2018
(51) Int. Cl.: G16H 10/00

(54) **METHOD AND SYSTEM FOR DISPLAYING CLINICAL INFORMATION ON A SCREEN OF A WEARABLE DEVICE**

(71) Applicant: Siemens Healthcare GmbH, 91052 Erlangen (DE)
(72) Inventor: Boettger, Thomas, 91054 Erlangen (DE); Hölzer, Philipp, Baltimore, 21201 (US); Lerch, Daniel, 91365 Weilersbach (DE); Thierfelder, Carsten, 91361 Pinzberg (DE)

(57) **Abstract**

Method for displaying clinical information on a screen of a wearable device, the method comprising the following steps:
- receiving clinical data with an interface,
- determining clinical information that is optimized for being displayed on the screen of the wearable device by applying a trained machine learning algorithm onto the clinical data with a calculation unit,
- displaying the clinical information on the screen of the wearable device.

## Description

The invention relates in one aspect to a method for displaying clinical information on a screen of a wearable device. The invention relates in a further aspect to a system for displaying clinical information on a screen of a wearable device. The invention relates in a further aspect to a training method for training a machine learning algorithm.

In healthcare it is crucial to observe and control patient conditions and clinical workflows. Therefore, plenty of monitoring devices are being used and a vast amount of information is generated. This information is typically displayed to operators, in a medical examination room and/or in a control room.

Typically, in the medical examination room and/or in the control room, several devices (computer displays, tablet-PCs, warning lights, loudspeakers for acoustic signals, ...) are present, which all display a certain specific type of information (medical images, patient data, x-ray warning light, warning buzzer, ...) simultaneously and leave it to the perception of the technologist to filter out the information which is currently the most relevant or crucial. This results in stress and sometimes missing the most important information, which in turn poses a risk to patient safety and well-being.

To allow for a more location independent on the spot information and reaction, it would be beneficial to provide such information to technologists and operators directly, e.g. by using wearable devices, for instance, smartwatches. Smartwatches hold a huge potential for various workflow aspects in clinical settings, e.g. location independent control of clinical workflow scenarios, immediate reaction to critical events or on the spot communication with other clinical personnel, supervisors, etc. Outside of medical imaging, smartwatches have gained popularity as compact and mobile general purpose computers.

Example functionalities include those of a calculator, time teller, camera, accelerometer, thermometer, digital map, scheduler, personal organizer, phone, etc. In the context of medical imaging they can be used as an alternative means of retrieving, visualizing or processing patient data such as electronic medical record, imaging data, lab data, pathology data, patient history, etc.

Up to now smartwatches have been used that show the same clinical content as other display devices. However, in contrast to monitors, laptops, tablets, etc. the display of smartwatches tends to be very small so that data either has to be resized beyond readability or content has to be pre-selected for either display or discard.

US 2015/0097701 A1 discloses a system for providing medical data translation for output on a medical monitoring hub.

US 2015/0364022 A1 discloses an apparatus for modifying alarms at a medical device.

US 2017/0039822 A1 discloses a dynamic alarm system.

US 2018/0060489 A1 discloses a method for outputting an item of medical information concerning at least one of a suitability of a patient for an examination via a medical imaging device and a patient-specific configuration of a medical imaging device.

The underlying technical problem of the invention is to facilitate an improved display of clinical information on a screen of a wearable device. This problem is solved by the method of claim 1, by the system of claim 8, and by the training method of claim 15. The dependent claims are related to further aspects of the invention.

In one aspect the invention relates to a method for displaying clinical information on a screen of a wearable device, the method comprising the following steps:
- receiving clinical data with an interface,
- determining clinical information that is optimized for being displayed on the screen of the wearable device by applying a trained machine learning algorithm onto the clinical data with a calculation unit,
- displaying the clinical information on the screen of the wearable device.

It is proposed to use a smartwatch in conjunction with a deep learning algorithm that automatically determines and displays the most important content of clinical information. The clinical data can be received, for example, by receiving a signal that carries the clinical data and/or by reading the data from a computer-readable medium.

In a further aspect the invention relates to a method for displaying clinical information on a screen of a smartwatch, the method comprising the following steps:
- receiving clinical data with an interface,
- determining clinical information that is optimized for being displayed on the screen of the smartwatch by applying a trained machine learning algorithm onto the clinical data with a calculation unit,
- displaying the clinical information on the screen of the smartwatch.

In another aspect the method further comprises the following step:
- monitoring a patient with a patient monitoring system, thereby obtaining patient monitoring data,
- wherein the clinical data comprises the patient monitoring data.

The patient monitoring system may comprise several detectors of different types (optical, motion, acoustic, physiological) that can be combined in order become aware of certain changes in the patient condition that require the attention of the technologist. This is because the clinical processes and workflows often require the operator to leave the patient unattended on the medical device.

The patient monitoring data may comprise optical image data of the patient and/or of certain regions of the patient body, for example the face. The trained machine learning algorithm may be configured to detect stress or pain in the face or posture of the patient. This is particular important in case of a sudden seizure or allergic reactions to contrast media or extravasation during contrast media injection. This would result in harm to patient safety and wellbeing if not noticed by operator

The trained machine learning algorithm may be configured to detect patient motion after he has been positioned, which would be detrimental to the imaging result if not noticed and corrected by operator.
The trained machine learning algorithm may be configured to detect that patient is calling or asking questions. If nobody answers because this goes unnoticed, this can result in anything from mild anxiety (higher breathing rates and thus motion artifacts in images) over motion of the patient in order to see where operator has gone (bad positioning, see above) to severe patient stress, resulting in paranoia or claustrophobia resulting in a refusal to take the exam.

In another aspect the method further comprises the following step:
- monitoring an operator with an operator monitoring system, thereby obtaining operator monitoring data,
- wherein the clinical data comprises the operator monitoring data.

The operator monitoring data may comprise data related to the location and/or the stress condition of the operator. The operator may be an operator of the medical imaging device. The location of the operator can be used to modify the reaction of the trained machine learning algorithm to certain input conditions. Furthermore todays standard functions of smartwatches, i.e. physiological measurements of heart rate, transportation etc. can be used to monitor stress conditions of the at least one patient and/or the at least one operator wearing the corresponding smartwatch. This yields new opportunities to detect difficult situations much earlier.

For example, if an event leads to critical patient conditions and the operators do not respond to it, the device - the smartwatch could - raise additional awareness by triggering an alarm. In addition such smartwatch will be a networked device and would also allow for triggering additional alerts remotely, e.g. for sending assistance.

In all of the above the algorithm could keep track of the input from these detectors and determine whether any of them needs the awareness and reaction from the operator. In case of multiple abnormalities, the algorithm will sort these findings according to criticality. These or the most critical input can then be forwarded in a situation-dependent via the smartwatch to the operator, which is able to reach the operator in any location where the Smartwatch has connectivity.

In another aspect the clinical data comprises at least one of patient history data, patient electronic medical record data, patient laboratory data, data provided by a patient monitoring system, patient conditions, data provided by an operator monitoring system operator location, operator conditions, treatment conditions, clinical pathway data, treatment history, medical image data, and medical imaging device configuration data. Data provided by the patient monitoring system and/or by the operator monitoring system can comprise, for example, at least one of infrared camera data, 2D camera data, 3D camera data, radar data, navigational data (e. g. GPS data), electrocardiography (ECG) data, pulse oximetry data, blood pressure data, respiratory data and activity tracker data.

In another aspect the clinical data comprises a plurality of subsets of the clinical data, wherein the trained machine learning algorithm is configured to determine a measure of importance for each of the subsets of the plurality of subsets, to select a subset from the plurality of subsets based on the measure of importance and to process the selected subset, thereby obtaining the clinical information that is optimized for being displayed on the screen of the wearable device.

The trained machine learning algorithm may be configured to automatically determine a region of abnormality and/or pathology in a medical image. The clinical information may comprise an image of this region, so that only this region is displayed on the screen of the wearable device.

The trained machine learning algorithm may be configured to automatically determine an appropriate view in a 3D-rendered image and/or in a cinematically rendered image, so that the appropriate view is displayed as part of the clinical information on the screen of the wearable device.

The trained machine learning algorithm may be configured to automatically determine the best spectral parameter (appropriate level of mono-energetic energy, virtual-non-contrast vs iodine map, etc.) for display of a spectrally resolved CT and/or radiography image.

Furthermore, depending on the clinical question, post-processing routines such as bone removal can be used by the trained machine learning algorithm to provide post-processed medical images as part of the clinical information displayed on the screen of the wearable device.

The trained machine learning algorithm may be configured to automatically determine the most important subset of patient data, which is relevant during the current workflow step.

This can be e.g. name and photo of the patient when next patient is due for registration, clinical indication during patient registration, lab values (needed to assess kidney function) while preparing a registered patient for contrast examinations. Additionally, the algorithm can also evaluate other data such as lab data, pathology data, functional tests, electronic medical records, etc. For example, if the image-based finding is a suspicious lung nodule, additional relevant data that can be displayed might be smoking history of patient, genetic predisposition, etc.

In case of multiple patient data, the trained machine learning algorithm may be configured to sort these image series according to urgency so that the most critical patient will be shown first and through touch-based scrolling data of other patients can be displayed.

Further aspects regarding optimized information representation on small wearable devices could be:
- Situation dependent user interfaces, e.g. a patient collapse mode for the user interface of the wearable device,
- Adaptive content based on trends, e.g. once a critical event has been detected, automatically switch displayed content to next state, e.g. decision point visualization: send to emergency room vs. send to operating room,
- Automatically call emergency room physician on duty, and
- Dispatch nurse request.

Images can be acquired by one or a combination of the following modalities: CT, SPECT, PET, MRI, Ultrasound, x-ray radiography, photoacoustic imaging, magnetic particle imaging, optical coherence tomography, optical camera, infrared camera, 3d camera/ depth camera, endoscopy, digital holographic microscopy, etc. Images can be multidimensional such as photon counting, multispectral for CT, multiple sequences for MRI, etc. Images can be functional, e.g. Perfusion. Images can be conformant to DICOM standard. Image processing can be done locally or remotely using cloud computing, edge computing, fog computing.

Visualization on the smartwatch can include the following: lung nodules, ca scores, FFR values, risk of plaque rupture, clots/emboli, stenosis, bone density, radiomic features, texture features, bleeding, aneurysms, vessel diameters, vessel wall thicknesses, vessel tapering indices, airway diameters, airway wall thicknesses, airway tapering indices, emphysema volume, vertebral compression fractures, gas trapping, liver fat, epicardial fat, iron concentration, fat concentration, bone fractures, etc.

In another aspect the clinical data comprises data regarding a plurality of potential receivers of the clinical information,
- wherein the trained machine learning algorithm predicts an optimal receiver for the clinical information from the plurality of potential receivers of the clinical information,
- wherein the clinical information is transmitted to the wearable device of the predicted optimal receiver, for example, by a network,
- wherein the clinical information is displayed on the screen of the wearable device of the predicted optimal receiver.

If the clinical data comprises an image that shows a finding that requires immediate action, a surgeon or interventionalist wearing such smartwatch could be alerted. In order to enable a fast decision only content that has been pre-selected by the trained machine learning algorithm will be shown to the surgeon or interventionalist.
Furthermore depending on the criticality of the detected input to be forwarded to the operator as well as the location of the operator and/or the wearable device of the receiver relative to the medical device, the algorithm can determine how to provide the warning signal in the most appropriate way to the operator.

If the operator is close to the patient and danger is mild, the information to the operator should be as subtle as possible to catch the operator's intention in order not to disturb the patient tranquility. That means e.g. in case of severe danger for patient safety or health and far distance of the operator from the patient the smartwatch can emit a strong vibration accompanied with acoustic and/or optical signals to make sure the attention of the operator is caught immediately independent of the surrounding level of noise or distraction.

If the patient is detected to be in need of interaction (for example, calls for help or moves in agony) while the operator is too far to answer directly, the system could play an automatic voice message to calm the patient down and inform him that help is on the way while the operator is called via smartwatch.

An artificial intelligence module capable of natural language processing could analyze the verbal input via speech recognition and formulate a suitable answer to the patient's questions while the operator is called. In another aspect, a direct communication channel could be opened between operator and patient (via microphone and loudspeaker on smartwatch and medical device) to enable direct and immediate communication to the patient.

In another aspect the wearable device is a smartwatch. The wearable device comprises a screen, in particular a touchscreen. The screen of the wearable device may be of limited size compared to the screen of a touchpad. In particular, the diagonal of the screen of the wearable device may be smaller than three inches, preferably smaller than two inches, for example smaller than 1.5 inches.

In another aspect, the wearable device comprises a bracelet and/or a watchstrap and/or other means of attaching the wearable device to a human body part. The human body part can be, for example, a wrist, a forearm, an upper arm, a neck or a chest.

In a further aspect the invention relates to a system for displaying clinical information on a screen of a wearable device, the system comprising the following units:
- interface, configured for receiving clinical data,
- calculation unit, configured for determining clinical information that is optimized for being displayed on the screen of the wearable device by applying a trained machine learning algorithm,
- wearable device comprising a screen, configured for displaying the clinical information on the screen of the wearable device.

In another aspect, the wearable device comprises the interface and/or the calculation unit. In another aspect, the interface and/or the calculation unit is separate to the wearable device.

The clinical information may be transmitted from the calculation unit to the wearable device and/or to the screen of the wearable device, for example, by a network and/or by a signal that carries the clinical information.

In another aspect the system further comprises:
- a patient monitoring system, configured for monitoring at least one patient, thereby obtaining patient monitoring data,
- wherein the clinical data comprises the patient monitoring data.

In another aspect the system is the wearable device, for example, a smartwatch. In a further aspect the invention relates to a wearable device for displaying clinical information on a screen of the wearable device, the wearable device comprising the following units:
- interface, configured for receiving clinical data,
- calculation unit, configured for determining clinical information that is optimized for being displayed on the screen of the wearable device by applying a trained machine learning algorithm,
- a screen, configured for displaying the clinical information on the screen of the wearable device.

In another aspect the system further comprises:
- an operator monitoring system, configured for monitoring at least one operator, thereby obtaining operator monitoring data,
- wherein the clinical data comprises the operator monitoring data.

In another aspect the clinical data comprises at least one of patient history data, patient electronic medical record data, clinical pathway data, laboratory data, medical image data, and medical imaging device configuration data.

In another aspect the clinical data comprises a plurality of subsets of the clinical data, wherein the trained machine learning algorithm is configured to determine a measure of importance for each of the subsets of the plurality of subsets, to select a subset from the plurality of subsets based on the measure of importance and to process the selected subset, thereby obtaining the clinical information that is optimized for being displayed on the screen of the wearable device.

In another aspect the clinical data comprises data regarding a plurality of potential receivers of the clinical information,
- wherein the trained machine learning algorithm is configured to predict an optimal receiver for the clinical information from the plurality of potential receivers of the clinical information.

In another aspect, the system is configured to transmit the clinical information to the wearable device of the predicted optimal receiver, for example, by a network, and to display the clinical information on the screen of the wearable device of the predicted optimal receiver.

Each of the potential receivers of the clinical information may comprise a wearable device, for example, a smartwatch.

In a further aspect the invention relates to a training method for training a machine learning algorithm, the training method comprising the following steps:
- providing training data, the training data comprising a plurality of training samples,
- wherein each training sample of the plurality of training samples comprises a clinical data sample,
- wherein each training sample of the plurality of training samples further comprises a clinical information sample that was determined based on the corresponding clinical data sample and served as a basis for a clinical pathway decision and/or a clinical pathway decision sample that was determined based on the corresponding clinical data sample and/or the corresponding clinical information sample,
- training the machine learning algorithm based on the training data, thereby obtaining a trained machine learning algorithm configured for determining clinical information that is optimized for being displayed on the screen of a wearable device by applying the trained machine learning algorithm onto clinical data with a calculation unit.

In another aspect, each training sample of the plurality of training samples may further comprise a measure of examination and/or treatment success in relation to the corresponding clinical information sample and/or the corresponding clinical pathway decision.

The training data may comprise one or more of the following elements: patient history, patient's electronic medical record, measurements from available physiological sensors, e.g. ECG, patient conditions, operator location, operator conditions, treatment conditions, selected treatment pathway, treatment history and longtime patient survival rate, and history of treatment success in relation to clinical pathway decisions.

The trained machine learning algorithm may be configured to perform one or more of the following steps:
- run a deep learning detection routine on a medical image to detect, for example, an abnormality,
- selecting of a region to be displayed as a part of the clinical information on the screen of the wearable device.
- modify the medical image by subsequently covering a limited area within the image (in the simplest case, covering up a part of the image and shifting this blanking filter across the image),
- comparing the output of both readings, wherein the region to be displayed on the smartwatch is determined as the region corresponding to a filter position where there is the greatest discrepancy in the output between the untouched and the modified image,
- segmenting and/or contouring a structure in the medical image, and
- evaluating and/or labeling a structure in the medical image.

The trained machine learning algorithm can include one or more of the following modules:
- Training data,
- at least one learning-based evaluation algorithm, and
- at least one prediction model.

The trained machine learning algorithm may comprise a prediction model for predicting the importance of a subset of the clinical data and/or a prediction model for predicting an optimal receiver of the clinical information.

The trained machine learning algorithm can be based on one or more of the following architectures: convolutional neural networks, deep belief networks, deep residual learning, deep reinforcement learning, recurrent neural networks, Siamese networks, generative adversarial networks, or auto-encoders. In particular, the trained machine learning algorithm can be a deep learning algorithm.

Alternatively, other machine learning implementations based on support vector machines, Bayesian classifiers, k-means clustering, decision trees, or inductive programming might also be conceivable.

According to one embodiment, the system for displaying clinical information on a screen of a wearable device comprises one or more of the following components:
- a calculation unit configured to execute a deep learning algorithm for the selection of most relevant content as described above,
- a smartwatch containing one or more of the following features: a display (such as OLED, LCD, or hologram) that contains touch sensitive technology as described below, a watchstrap or other means of attaching the smartwatch to a human body part such as a wrist, a forearm, an upper arm, a neck or a chest, an operating system, a data encryption system, a microphone or speaker system, a natural language processing system, a light projector, a linear resonant actuator or vibration motor for haptic feedback, and a camera,
- means for wireless communication (through electromagnetic or acoustic waves) with a medical device such as a medical imaging modality, a PACS system, a workstation for diagnostic reading, a camera (e.g. attached to the scanner for positioning or patient monitoring).

The screen of the wearable device can be a touchscreen. The touchscreen of the wearable device may be based on one or more of the following technologies.

A resistive touchscreen panel comprises several thin layers, the most important of which are two transparent electrically resistive layers facing each other with a thin gap between. The top layer (that which is touched) has a coating on the underside surface; just beneath it is a similar resistive layer on top of its substrate. One layer has conductive connections along its sides, the other along top and bottom. A voltage is applied to one layer, and sensed by the other. When an object, such as a fingertip or stylus tip, presses down onto the outer surface, the two layers touch to become connected at that point. The panel then behaves as a pair of voltage dividers, one axis at a time. By rapidly switching between each layer, the position of pressure on the screen can be determined.

Resistive touch is used in restaurants, factories and hospitals due to its high tolerance for liquids and contaminants. A major benefit of resistive-touch technology is its low cost. Additionally, as only sufficient pressure is necessary for the touch to be sensed, they may be used with gloves on, or by using anything rigid as a finger substitute. Disadvantages include the need to press down and a risk of damage by sharp objects. Resistive touchscreens also suffer from poorer contrast, due to having additional reflections (i.e.: glare) from the layers of material placed over the screen.

Surface acoustic wave (SAW) technology uses ultrasonic waves that pass over the touchscreen panel. When the panel is touched, a portion of the wave is absorbed. The change in ultrasonic waves is processed by the controller to determine the position of the touch event. Surface acoustic wave touchscreen panels can be damaged by outside elements. Contaminants on the surface can also interfere with the functionality of the touchscreen.

A capacitive touchscreen panel consists of an insulator, such as glass, coated with a transparent conductor, such as indium tin oxide (ITO). As the human body is also an electrical conductor, touching the surface of the screen results in a distortion of the screen's electrostatic field, measurable as a change in capacitance. Different technologies may be used to determine the location of the touch. The location is then sent to the controller for processing.

Unlike a resistive touchscreen, one cannot use a capacitive touchscreen through most types of electrically insulating material, such as gloves. This disadvantage especially affects usability in consumer electronics, such as touch tablet PCs and capacitive smartphones, in cold weather. It can be overcome with a special capacitive stylus, or a special-application glove with an embroidered patch of conductive thread allowing electrical contact with the user's fingertip.

Leading capacitive display manufacturers continue to develop thinner and more-accurate touchscreens. Those for mobile devices are now being produced with 'in-cell' technology that eliminates a layer by building the capacitors inside the display itself. This type of touchscreen reduces the visible distance between the user's finger and what the user is touching on the screen, creating a more-direct contact with the image of displayed content and enabling taps and gestures to be more responsive.

A simple parallel-plate capacitor has two conductors separated by a dielectric layer. Most of the energy in this system is concentrated directly between the plates. Some of the energy spills over into the area outside the plates, and the electric field lines associated with this effect are called fringing fields. Part of the challenge of making a practical capacitive sensor is to design a set of printed circuit traces which direct fringing fields into an active sensing area accessible to a user. A parallel-plate capacitor is not a good choice for such a sensor pattern. Placing a finger near fringing electric fields adds conductive surface area to the capacitive system. The additional charge storage capacity added by the finger is known as finger capacitance, or CF. The capacitance of the sensor without a finger present is known as parasitic capacitance, or CP.

In this basic technology, only one side of the insulator is coated with a conductive layer. A small voltage is applied to the layer, resulting in a uniform electrostatic field. When a conductor, such as a human finger, touches the uncoated surface, a capacitor is dynamically formed. The sensor's controller can determine the location of the touch indirectly from the change in the capacitance as measured from the four corners of the panel. As it has no moving parts, it is moderately durable but has limited resolution, is prone to false signals from parasitic capacitive coupling, and needs calibration during manufacture. It is therefore most often used in simple applications such as industrial controls and kiosks.

Projected capacitive touch (PCT; also PCAP) technology is a variant of capacitive touch technology. All PCT touch screens are made of a matrix of rows and columns of conductive material, layered on sheets of glass. This can be done either by etching a single conductive layer to form a grid pattern of electrodes, or by etching two separate, perpendicular layers of conductive material with parallel lines or tracks to form a grid. Voltage applied to this grid creates a uniform electrostatic field, which can be measured. When a conductive object, such as a finger, comes into contact with a PCT panel, it distorts the local electrostatic field at that point. This is measurable as a change in capacitance. If a finger bridges the gap between two of the "tracks", the charge field is further interrupted and detected by the controller. The capacitance can be changed and measured at every individual point on the grid. This system is able to accurately track touches. Due to the top layer of a PCT being glass, it is sturdier than less-expensive resistive touch technology. Unlike traditional capacitive touch technology, it is possible for a PCT system to sense a passive stylus or gloved finger. However, moisture on the surface of the panel, high humidity, or collected dust can interfere with performance. There are two types of PCT: mutual capacitance and self-capacitance.

This is a common PCT approach, which makes use of the fact that most conductive objects are able to hold a charge if they are very close together. In mutual capacitive sensors, a capacitor is inherently formed by the row trace and column trace at each intersection of the grid. A 16x14 array, for example, would have 224 independent capacitors. A voltage is applied to the rows or columns. Bringing a finger or conductive stylus close to the surface of the sensor changes the local electrostatic field, which in turn reduces the mutual capacitance. The capacitance change at every individual point on the grid can be measured to accurately determine the touch location by measuring the voltage in the other axis. Mutual capacitance allows multi-touch operation where multiple fingers, palms or styli can be accurately tracked at the same time.

Self-capacitance sensors can have the same X-Y grid as mutual capacitance sensors, but the columns and rows operate independently. With self-capacitance, the capacitive load of a finger is measured on each column or row electrode by a current meter. This method produces a stronger signal than mutual capacitance, but it is unable to accurately resolve more than one finger, which results in "ghosting" or misplaced location sensing.

Capacitive touchscreens do not necessarily need to be operated by a finger, but until recently the special styli required could be quite expensive to purchase. The cost of this technology has fallen greatly in recent years and capacitive styli are now widely available for a nominal charge, and often given away free with mobile accessories.

An infrared touchscreen uses an array of X-Y infrared LED and photodetector pairs around the edges of the screen to detect a disruption in the pattern of LED beams. These LED beams cross each other in vertical and horizontal patterns. This helps the sensors pick up the exact location of the touch. A major benefit of such a system is that it can detect essentially any opaque object including a finger, gloved finger, stylus or pen. It is generally used in outdoor applications and POS systems which cannot rely on a conductor (such as a bare finger) to activate the touchscreen. Unlike capacitive touchscreens, infrared touchscreens do not require any patterning on the glass which increases durability and optical clarity of the overall system. Infrared touchscreens are sensitive to dirt and dust that can interfere with the infrared beams, and suffer from parallax in curved surfaces and accidental press when the user hovers a finger over the screen while searching for the item to be selected.

A translucent acrylic sheet is used as a rear-projection screen to display information. The edges of the acrylic sheet are illuminated by infrared LEDs, and infrared cameras are focused on the back of the sheet. Objects placed on the sheet are detectable by the cameras. When the sheet is touched by the user the deformation results in leakage of infrared light which peaks at the points of maximum pressure, indicating the users touch location.

Optical touchscreens are a relatively modern development in touchscreen technology, in which two or more image sensors are placed around the edges (mostly the corners) of the screen. Infrared backlights are placed in the camera's field of view on the opposite side of the screen. A touch blocks some lights from the cameras, and the location and size of the touching object can be calculated (see visual hull). This technology is growing in popularity due to its scalability, versatility, and affordability for larger touchscreens.

This system detects a touch by using sensors to measure the piezoelectricity in the glass. Complex algorithms interpret this information and provide the actual location of the touch. The technology is unaffected by dust and other outside elements, including scratches. Since there is no need for additional elements on screen, it also claims to provide excellent optical clarity. Any object can be used to generate touch events, including gloved fingers. A downside is that after the initial touch, the system cannot detect a motionless finger. However, for the same reason, resting objects do not disrupt touch recognition.

The key to this technology is that a touch at any one position on the surface generates a sound wave in the substrate which then produces a unique combined signal as measured by three or more tiny transducers attached to the edges of the touchscreen. The digitized signal is compared to a list corresponding to every position on the surface, determining the touch location. A moving touch is tracked by rapid repetition of this process. Extraneous and ambient sounds are ignored since they do not match any stored sound profile. The technology differs from other sound-based technologies by using a simple look-up method rather than expensive signal-processing hardware. As with the dispersive signal technology system, a motionless finger cannot be detected after the initial touch. However, for the same reason, the touch recognition is not disrupted by any resting objects.

The technology is described by the patent family EP1852772. The touchscreens based on this technology can be made of ordinary glass, giving good durability and optical clarity. The technology usually retains accuracy with scratches and dust on the screen. The technology is also well suited to displays that are physically larger.

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention

Reference is made to the fact that the described methods and the described system are merely preferred example embodiments of the invention and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a diagram illustrating a method for displaying clinical information on a screen of a wearable device.
Fig. 2 shows a diagram illustrating another example embodiment of a method for displaying clinical information on a screen of a wearable device.
Fig. 3 shows a system for displaying clinical information on a screen of a wearable device.
Fig. 4 shows another example embodiment of a system for displaying clinical information on a screen of a wearable device.
Fig. 5 shows a system for displaying clinical information on a screen of a wearable device, the system comprising a medical imaging device.
Fig. 6 shows a diagram illustrating a training method for displaying clinical information on a screen of a wearable device.

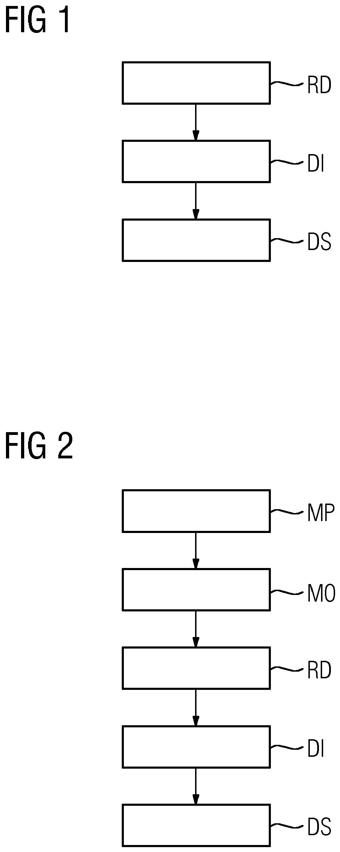

Fig. 1 shows a diagram illustrating a method for displaying clinical information on a screen S of a wearable device W1, the method comprising the following steps:
   - receiving RD clinical data with an interface IF,
   - determining DI clinical information that is optimized for being displayed on the screen of the wearable device by applying a trained machine learning algorithm onto the clinical data with a calculation unit CU,
   - displaying DS the clinical information on the screen S of the wearable device W1.
Fig. 2 shows a diagram illustrating another example embodiment of a method for displaying clinical information on a screen S of a wearable device W1, the method further comprising the following steps:
   - monitoring MP at least one patient 13 with a patient monitoring system PMS, thereby obtaining patient monitoring data,
   - monitoring MO at least one operator U1, U2 with an operator monitoring system OMS, thereby obtaining operator monitoring data,
   - wherein the clinical data comprises the patient monitoring data and the operator monitoring data.

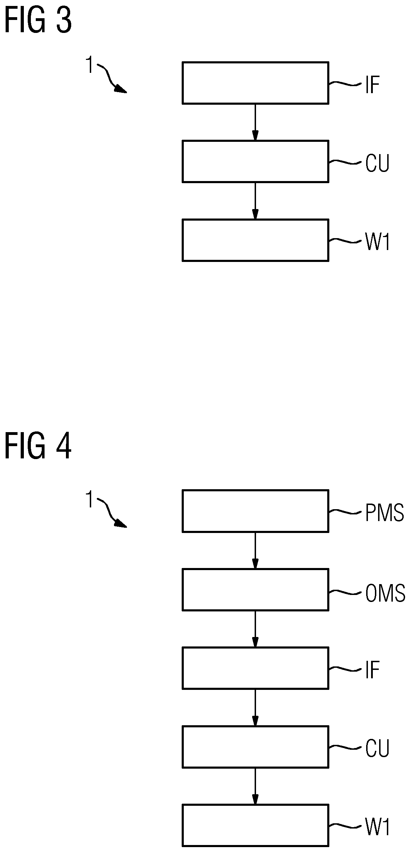

Fig. 3 shows a system 1 for displaying clinical information on a screen S of a wearable device W1, the system 1 comprising the following units:
   - interface IF, configured for receiving clinical data,
   - calculation unit CU, configured for determining clinical information that is optimized for being displayed on the screen S of the wearable device W1 by applying a trained machine learning algorithm onto the clinical data,
   - the wearable device W1 comprising the screen S, wherein the wearable device W1 is configured for displaying the clinical information on the screen S of the wearable device W1.
Fig. 4 shows another example embodiment of a system for displaying clinical information on a screen S of a wearable device W1, the system 1 further comprising:
   - a patient monitoring system PMS, configured for monitoring a patient 13, thereby obtaining patient monitoring data,
   - an operator monitoring system OMS, configured for monitoring an operator U1, thereby obtaining operator monitoring data,
   - wherein the clinical data comprises the patient monitoring data and the operator monitoring data.

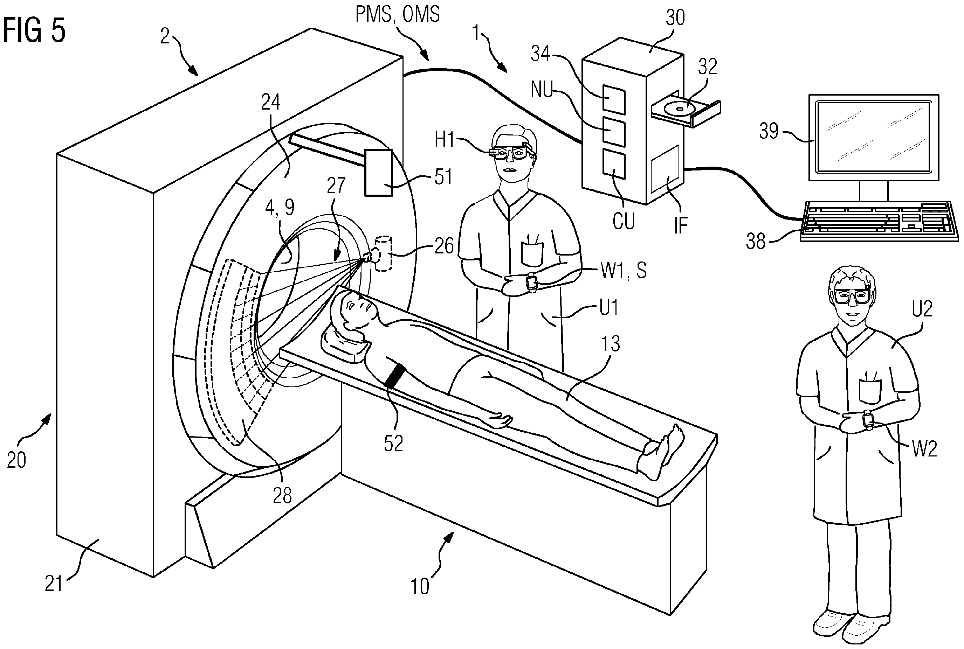

Fig. 5 shows a system 1 for displaying clinical information on a screen S of a wearable device W1, the system 1 comprising a medical imaging device 2. The medical imaging device 2 is a computed tomography device. The system 1 comprises two wearable devices W1 and W2 in form of smartwatches. The smartwatch W1 is worn by a first operator U1, for example a physician. The smartwatch W2 is worn by a second operator U2, for example a medical assistant. The first operator U1 and the second operator U2 both are potential receivers of the clinical information.

The clinical data comprises data regarding a plurality of potential receivers of the clinical information, the plurality or potential receivers including the first operator U1 and the second operator U2. The trained machine learning algorithm is configured to predict an optimal receiver for the clinical information from the plurality of potential receivers of the clinical information. The clinical information can be displayed on the screen S of the wearable device W1 or W2 of the predicted optimal receiver.

The system further comprises a 2D and/or 3D optical camera 51, a wearable device 52 in form of a bracelet configured for tracking physiological data of the patient 13 and a wearable device H1 in form of data glasses worn by the first operator U1. The patient monitoring system PMS comprises the optical camera 51, the bracelet 52 and the data glasses H1. The operator monitoring system OMS comprises the optical camera 51, the data glasses H1 and the wearable devices W1 and W2.

The system 1 further comprises a control unit 30, the control unit 30 comprising the computer-readable medium 32, the image reconstruction unit 34, the interface IF, the calculating unit CU and the network unit NU.

The interface IF can be embodied as a hardware interface and/or as a software interface (e.g. PCI-Bus, USB or Fire-wire). In general, a calculation unit CU can comprise hardware elements and software elements, for example a microprocessor, a field programmable gate array (an acronym is "FPGA") or an application specific integrated circuit (an acronym is "ASIC"). A computer-readable medium 32 can be embodied as non-permanent main memory (e.g. random access memory) or as permanent mass storage (e.g. hard disk, USB stick, SD card, solid state disk). The calculation unit CU and/or the computer-readable medium 32 can be embodied in form of a data processing system based on distributed and/or cloud computing.

The wearable devices W1, W2, 52 and HI, the optical camera 51 and the medical imaging device 2 can communicate with each other via a network. The network can be realized as a LAN (acronym for "local area network"), in particular a WiFi network, or any other local connection, e.g. via Bluetooth or USB (acronym for "universal serial bus"). The network can also be realized as a WAN (acronym for "wide area network"); in particular the network can be identical with the internet. The network can alternatively also be realized as a VPN (acronym for "virtual private network"). The network unit NU is configured for providing the network.

The computed tomography device 2 comprises a gantry 20 with a support frame 21 and a rotating frame 24. An x-ray source 26 and an x-ray detector 28 are mounted on the rotating frame 24 for rotation around an acquisition portion 4 located within a tunnel-shaped opening 9. The computed tomography device 2 further comprises a patient handling system 10 for movably supporting the patient 13 such that at least a part of the patient body can be placed in the acquisition portion 4 in the tunnel-shaped opening 9 for penetration by x-rays 27. The input unit 38 can comprise means for inputting data (e.g. a keyboard or a mouse). The output unit 39 can comprise means for outputting data (e.g. a display or a printer).

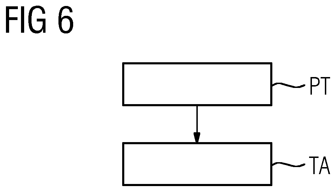

Fig. 6 shows a diagram illustrating a training method for displaying clinical information on a screen S of a wearable device W1, the training method comprising the following steps:
- providing PT training data, the training data comprising a plurality of training samples,
- wherein each training sample of the plurality of training samples comprises a clinical data sample,
- wherein each training sample of the plurality of training samples further comprises a clinical information sample that was determined based on the corresponding clinical data sample and served as a basis for a clinical pathway decision and/or a clinical pathway decision sample that was determined based on the corresponding clinical data sample and/or the corresponding clinical information sample,
- training TA the machine learning algorithm based on the training data, thereby obtaining a trained machine learning algorithm configured for determining clinical information that is optimized for being displayed on the screen S of a wearable device W1 by applying the trained machine learning algorithm onto clinical data with a calculation unit CU.

## Claims

1. Method for displaying clinical information on a screen (S) of a wearable device (W1), the method comprising the following steps:
- receiving (RD) clinical data with an interface (IF),
- determining (DI) clinical information that is optimized for being displayed on the screen (S) of the wearable device (W1) by applying a trained machine learning algorithm onto the clinical data with a calculation unit (CU),
- displaying (DS) the clinical information on the screen (S) of the wearable device (W1).

2. The method according to claim 1, the method further comprising the following step:
- monitoring (MP) at least one patient (13) with a patient monitoring system (PMS), thereby obtaining patient monitoring data,
- wherein the clinical data comprises the patient monitoring data.

3. The method according to claim 1 or 2, the method further comprising the following step:
- monitoring (MO) at least one operator (U1, U2) with an operator monitoring system (OMS), thereby obtaining operator monitoring data,
- wherein the clinical data comprises the operator monitoring data.

4. The method according to one of the claims 1 to 3,
- wherein the clinical data comprises at least one of patient history data, patient electronic medical record data, clinical pathway data, laboratory data, medical image data, and medical imaging device configuration data.

5. The method according to one of the claims 1 to 4,
- wherein the clinical data comprises a plurality of subsets of the clinical data,
- wherein the trained machine learning algorithm is configured to determine a measure of importance for each of the subsets of the plurality of subsets, to select a subset from the plurality of subsets based on the measure of importance and to process the selected subset, thereby obtaining the clinical information that is optimized for being displayed on the screen (S) of the wearable device (W1).

6. The method according to one of the claims 1 to 5,
- wherein the clinical data comprises data regarding a plurality of potential receivers of the clinical information,
- wherein the trained machine learning algorithm predicts an optimal receiver for the clinical information from the plurality of potential receivers of the clinical information,
- wherein the clinical information is transmitted to the wearable device (W1) of the predicted optimal receiver,
- wherein the clinical information is displayed on the screen (S) of the wearable device (W1) of the predicted optimal receiver.

7. The method according to one of the claims 1 to 6, wherein the wearable device (W1) is a smartwatch.

8. System (1) for displaying clinical information on a screen (S) of a wearable device (W1), the system (1) comprising the following units:
- interface (IF), configured for receiving clinical data,
- calculation unit (CU), configured for determining clinical information that is optimized for being displayed on the screen (S) of the wearable device (W1) by applying a trained machine learning algorithm onto the clinical data,
- the wearable device (W1) comprising the screen (S), wherein the wearable device (W1) is configured for displaying the clinical information on the screen (S) of the wearable device (W1) .

9. The system (1) according to claim 8, the system (1) further comprising:
- a patient monitoring system (PMS), configured for monitoring a patient (13), thereby obtaining patient monitoring data,
- wherein the clinical data comprises the patient monitoring data.

10. The system (1) according to claim 8 or 9, the system (1) further comprising:
- an operator monitoring system (OMS), configured for monitoring an operator (U1), thereby obtaining operator monitoring data,
- wherein the clinical data comprises the operator monitoring data.

11. The system (1) according to one of the claims 8 to 10,
- wherein the clinical data comprises at least one of patient history data, patient electronic medical record data, clinical pathway data, laboratory data, medical image data, and medical imaging device configuration data.

12. The system (1) according to one of the claims 8 to 11,
- wherein the clinical data comprises a plurality of subsets of the clinical data,
- wherein the trained machine learning algorithm is configured to determine a measure of importance for each of the subsets of the plurality of subsets, to select a subset from the plurality of subsets based on the measure of importance and to process the selected subset, thereby obtaining the clinical information that is optimized for being displayed on the screen (S) of the wearable device (W1).

13. The system (1) according to one of the claims 8 to 12,
- wherein the clinical data comprises data regarding a plurality of potential receivers of the clinical information,
- wherein the trained machine learning algorithm is configured to predict an optimal receiver for the clinical information from the plurality of potential receivers of the clinical information,
- wherein the system (1) is configured to transmit the clinical information to the wearable device (W1) of the predicted optimal receiver and to display the clinical information on the screen (S) of the wearable device (W1) of the predicted optimal receiver.

14. The system (1) according to one of the claims 8 to 13, wherein the wearable device (W1) is a smartwatch.

15. A training method for training a machine learning algorithm, the training method comprising the following steps:
- providing (PT) training data, the training data comprising a plurality of training samples,
- wherein each training sample of the plurality of training samples comprises a clinical data sample,
- wherein each training sample of the plurality of training samples further comprises a clinical information sample that was determined based on the corresponding clinical data sample and served as a basis for a clinical pathway decision and/or a clinical pathway decision sample that was determined based on the corresponding clinical data sample and/or the corresponding clinical information sample,
- training (TA) the machine learning algorithm based on the training data, thereby obtaining a trained machine learning algorithm configured for determining clinical information that is optimized for being displayed on the screen (S) of a wearable device (W1) by applying the trained machine learning algorithm onto clinical data with a calculation unit (CU).
